(19) Europäisches Patentamt

European Patent Office

Office européen des brevets

(11) EP 1 533 647 A1

(12) **EUROPEAN PATENT APPLICATION**

(43) Date of publication:
25.05.2005 Bulletin 2005/21

(51) Int Cl.7: **G02B 27/44**, A61B 3/10

(21) Application number: **04105850.4**

(22) Date of filing: **17.11.2004**

| | |
|---|---|
| (84) Designated Contracting States: **AT BE BG CH CY CZ DE DK EE ES FI FR GB GR HU IE IS IT LI LU MC NL PL PT RO SE SI SK TR** Designated Extension States: **AL HR LT LV MK YU** | (72) Inventor: **Curatu, Eugene O.** **FL 32820, Orlando (US)** |
| (30) Priority: **24.11.2003 US 720422** | (74) Representative: **Hanna, Peter William Derek** **Hanna, Moore & Curley** **11 Mespil Road** **Dublin 4 (IE)** |
| (71) Applicant: **Alcon RefractiveHorizons, Inc.** **Fort Worth, Texas 76134 (US)** Designated Contracting States: **AT BE BG CH LI CY CZ DE DK EE ES FI FR GB GR HU IE IT LU MC NL PL PT RO SE SI SK TR** | |

(54) **Method and apparatus for aberroscope calibration and discrete compensation**

(57)     A calibration device (10,30) for an aberroscope is provided which includes an optical element (40,50) that is insertable into an optical path of a wavefront analyzer. The optical element is adapted to induce a predetermined aberration in a wavefront. The optical element, which may be transmissive or reflective, may comprise a lens optimized for a specific power and aberration; a computer-generated hologram; or a spatial light modulator. A substantially unaberrated wavefront (35) is passed along an optical path leading to a wavefront analyzer. A predetermined aberration is induced in the unaberrated wavefront to form an aberrated wavefront (37,37') using an optical element positioned in the optical path. The aberrated wavefront is then analyzed using the wavefront analyzer, which is calibrated using data generated by the wavefront analyzer from the aberrated wavefront.

Fig. 2

EP 1 533 647 A1

**Description**

**TECHNICAL FIELD OF THE INVENTION**

The present invention relates to optical measurement and correction systems and methods, and, more particularly, to corneal topography and ocular aberrations measurement and correction systems and methods.

**BACKGROUND OF THE INVENTION**

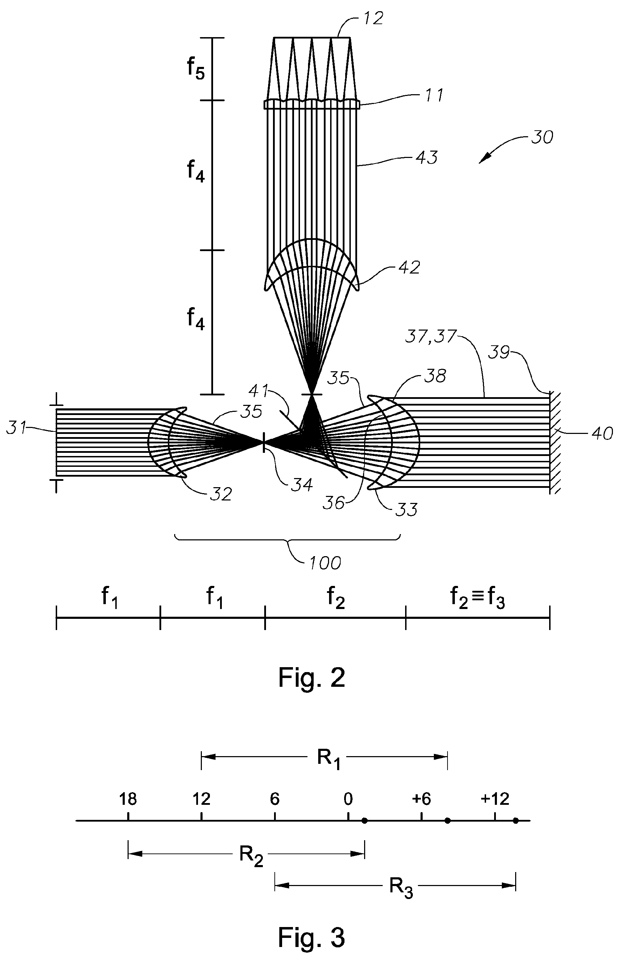

Wavefront measurement systems are known in the art for measuring and modeling ocular aberrations, such as those taught by Alcon Inc. (e.g., U.S. Pat. No. 6,271,914). This system and method uses Zernike polynomials to reconstruct an aberrated wavefront reflected from an eye and to calculate a desired profile for directing laser sculpting of the corneal surface. An exemplary schematic for such a wavefront measurement device is given in FIGURE 2 in the '914 patent.

Although a number of aberroscope designs are known in the art, calibration systems and methods are inadequate, as stated by the Optical Society of America Taskforce on Vision Science and Its Applications (VSIA-2000 and VSIA-2001). Calibration of wavefront analyzers is now typically performed at manufacturing sites, and not in the field. Further, classical lenses have primarily been used to provide a known amount of defocus by moving the lens back and forth in the optical path. This method has the disadvantages of being useful for limited aberration types (defocus, spherical aberration, and coma) and having a high level of uncertainty.

Holographic optical elements are known in the art that can function as lenses. Among their advantages are that they are lightweight and relatively inexpensive, can generate unique optical functions not possible with conventional optical elements, and can be fabricated in a wide range of materials.

Thus there is a need for a standard device that could be mass-produced for calibrating and validating aberrometers.

**BRIEF SUMMARY OF THE INVENTION**

The present invention provide a device, system, and method for calibrating an aberroscope, in accordance with claims which follow, such as, but not intended to be limited to, wavefront measurement devices for use in objective measurement of optical aberrations. The present invention also encompasses a method for making such a device and system.

An embodiment of the aberroscope calibration device of this invention comprises an optical element that is insertable into an optical path of a wavefront analyzer. The optical element is adapted to induce a predetermined aberration in a wavefront for presentation to the wavefront analyzer. Since the form of the aberration is known, the wavefront analyzer can be calibrated by comparing the predetermined aberration with an aberration calculated by the wavefront analyzer.

In specific embodiments, the optical element may comprise a lens optimized for a specific power and aberration; a computer-generated hologram, such as a diffractive optical element; or a spatial light modulator. The optical element may be transmissive or reflective.

A system for calibrating an aberroscope in accordance with this invention can comprise an optical element and a wavefront analyzer, the wavefront analyzer further comprising a wavefront detector. The wavefront detector is positioned at a downstream end of an optical path into which the optical element is placed.

A method for calibrating an aberroscope according to the teachings of this invention can comprise the steps of passing a substantially unaberrated wavefront along an optical path leading to a wavefront analyzer. A predetermined aberration is induced in the unaberrated wavefront to form an aberrated wavefront. The aberrated wavefront is induced by an optical element positioned in the optical path upstream of the wavefront analyzer. The aberrated wavefront exiting the optical element is analyzed by the wavefront analyzer. The wavefront analyzer is calibrated using data generated by the wavefront analyzer from the aberrated wavefront.

A method of constructing a device for calibrating an aberroscope according to the teachings of this invention can comprise the steps of determining a desired aberration and creating an optical element adapted to induce the desired aberration. The created optical element is positioned upstream of a wavefront analyzer to induce the desired aberration when it is desired to calibrate the wavefront analyzer.

The features that characterize the present invention, both as to organization and method of operation, together with further objects and advantages thereof, will be better understood from the following description taken in conjunction with the accompanying FIGUREs. It is to be expressly understood that the FIGUREs are for the purpose of illustration and description and are not intended as a definition of the limits of the invention. These and other objects attained, and advantages offered, by the present invention will become more fully apparent as the description that now follows is read in conjunction with the accompanying FIGUREs.

**BRIEF DESCRIPTION OF THE SEVERAL VIEWS OF THE DRAWINGS**

**FIGURE 1** is a schematic illustration of an exemplary aberrometer optical path in accordance with the teachings of the present invention.

**FIGURE 2** is a schematic illustration of an alternate embodiment of an aberrometer optical path.

**FIGURE 3** illustrates exemplary range shifts capable of being induced by an embodiment of the device of the present invention.

**FIGURES 4-7** are ray tracing graphs used in a paraxial approach to calculate the spot size at a lenslet array focal plane.

**FIGURE 8** illustrates a compound lens useful for inducing a known aberration in accordance with the present invention.

**DETAILED DESCRIPTION OF THE INVENTION**

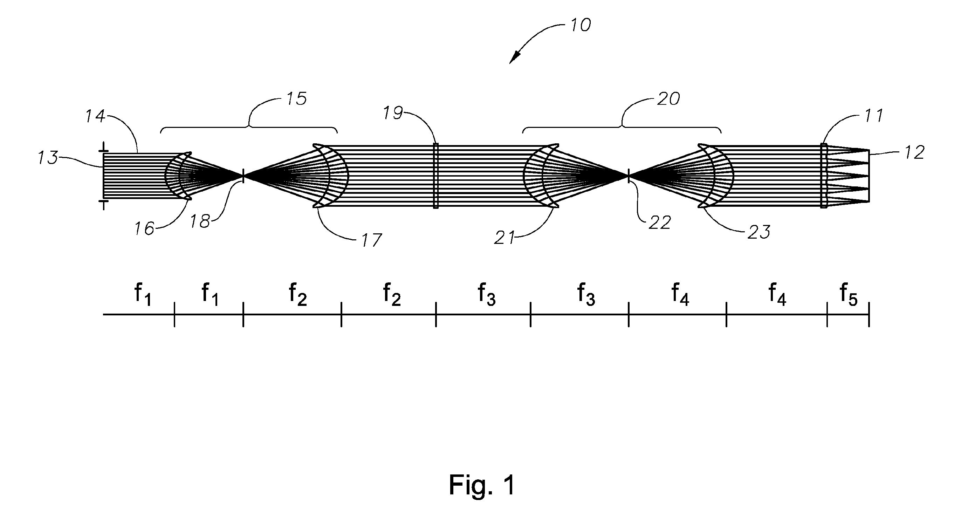

A description of the preferred embodiments of the present invention will now be presented with reference to FIGURES 1-8.

The method and system of the present invention comprise a plurality of embodiments for calibrating an aberrometer, for example, a wavefront analyzer, used to measure aberrations in an optical system. In a particular embodiment, the optical system comprises an eye, in which case the aberrometer is intended to measure optical aberrations preparatory to undertaking a corrective procedure, such as corneal ablation.

Embodiments of the system of the present invention comprise an optical element and a wavefront analyzer (aberrometer) for calibrating the wavefront analyzer. The wavefront analyzer may comprise, for example, a Hartmann-Shack wavefront sensor, although this is not intended as a limitation. In an exemplary embodiment illustrated in part in FIGURE 1, such a wavefront sensor comprises a lenslet array **11,** such as is known in the art, that samples a wavefront at regularly spaced points and transmits the sampled points onto a detector **12**.

In the case of a wavefront analyzer for use in measuring ocular optical aberrations, a wavefront reflected back from an eye contains data describing the eye's aberrations. As the measured aberrations are typically used to construct a prescription for a corrective procedure, it is important that the wavefront analyzer is calibrated so that an accurate prescription may be derived from the collected data.

An overarching principle of the present invention is therefore to provide a device and a method for inducing predetermined aberrations in a known wavefront, typically an unaberrated wavefront, so that a comparison of aberrations actually measured and calculated by the wavefront analyzer can be made with those that are theoretically expected from the predetermined aberrations. Adjustments can then be determined and made to the calculational process of the wavefront analyzer to compensate for any deviations from the expected measured results.

The optical train **10** of FIGURE 1 includes an entrance pupil **13** through which is admitted a wavefront **14** for analysis. A first afocal relay system **15** comprises, for example, a pair of lenses comprising a first focusing, or converging, lens **16**, and a first collimating lens **17**. First collimating lens **17** is positioned downstream of a first focal point **18** of the first focusing lens **16**. The first afocal relay system **15** images the source of the incoming wavefront **14** onto intermediate pupil plane **19**.

Downstream of the intermediate pupil plane **19** is positioned a second afocal relay system **20**, formed, as in the first afocal relay system **15**, by a pair of lenses comprising second focusing lens **21** having second focal point **22**, and second collimating lens **23**. The second afocal relay system **20** images the intermediate pupil plane **19** onto a lenslet array **11** plane. The lenslet array **11** samples the wavefront **14** in a number of wavelets, which impinge onto the detector **12** downstream of lenslet array **11**. Respective focal lengths $f_1$ - $f_5$ are shown below the optical train **10** of FIGURE 1.

An alternate architecture for an optical train **30** is illustrated in FIGURE 2, this architecture requiring fewer elements and a smaller footprint. The optical train **30** includes an entrance pupil **31**, and a first afocal relay system **100** comprising a pair of lenses. The lenses comprise a first focusing, or converging, lens **32,** and a first collimating lens **33**. First collimating lens **33** is positioned downstream of a first focal point **34** of the first focusing lens **32**. The first collimating lens **33** receives a wavefront **35** from the first converging lens **32** at a first face **36** and outputs a collimated wavefront **37** from a second face **38.**

The first afocal relay system **100** of FIGURE 2 images the entrance pupil **31** onto intermediate pupil plane **39.** A reflective optical element **40** is positioned at the intermediate pupil plane **39.** Between the lenses **32** and **33** is positioned a beamsplitter **41**, which may comprise a pellicle beamsplitter, through which the wavefront **35** exiting the first focusing lens **32** passes substantially unaltered.

The reflective optical element **40** serves to reflect the wavefront **37** exiting the first collimating lens **33** back through lens **33**, the path of the reflected wavefront **37'** now reversed, so that the first collimating lens **33** also serves as a second converging lens. The reflected wavefront **37'** is reflected by the beamsplitter **41** toward a second collimating lens **42,** where reflected wavefront **37'** exits second collimating lens **42** as wavefront **43**. Wavefront **43** impinges upon a lenslet array **11**. Focal lengths $f_1$ - $f_5$ are shown in FIGURE 2 alongside their respective optical path segments.

An optical train such as optical trains **10** and **30** described above, or another such optical train as can be conceived by one of average skill in the art, can be used by the method and system of this invention to calibrate an aberroscope, such as, but not intended to be limited to, a Hartmann-Shack analyzer, including a lenslet array **11** and detector **12**. In accordance with the teachings of this invention, such an aberroscope calibration can be performed using the optical element having a known aberration in the optical train **10** and/or **30**. The optical element, an Aberroscope Calibration Device ("ACD"), induces a change of phase to a wavefront passing through or reflected by the optical element, and has a phase surface modeled to reproduce a desired ocular wavefront for transforming a parallel beam into the desired wavefront. Such an optical element may comprise, but is not intended to be limited to, a lens optimized for a specific power and aberration; a diffractive optical element (DOE) or computer-generated hologram (CG H); or a spatial light modulator (SLM), such as a liquid crystal SLM, a Micro-Electro-Mechanical Systems (MEMS) device, or a continuous membrane deformable mirror. The optical element may be transmissive or reflective.

The optical element (ACD) may be inserted into a wavefront measurement system at any of the following locations: the entrance pupil plane; the intermediate pupil plane; or the lenslet array plane. In prior art systems, calibration of an Aberroscope is required to be performed at a manufacturing facility by introducing an aberrated wavefront at the entrance pupil plane. The embodiments of the present invention permit calibration to be performed at any time at an installed site without disturbing the Aberroscope installation. Further, the optical element of this invention may comprise a plurality of optical elements positionable one at a time in the optical train, such as, for example, by using a rotating turret holding the optical elements for serial insertion.

The ACD of the present invention can thus be used to perform calibration/validation of an instrument. For example, an ACD in a monochromatic collimated or diverging beam provides, with high accuracy, a wavefront containing a specific aberration (e.g., a Zernike spectrum). Although, theoretically, a phase function $\varphi(x,y)$ can be introduced by either a refractive or a diffractive element and can be described in different mathematical forms, it may be determined that a particular CGH aberrator may be more easily designed using a Zernike polynomial description.

Embodiments of the ACD of this invention can also be used to provide discrete compensation, "shifting" the origin of measurement by a known value. As an example (see FIGURE 3), if a given wavefront measurement instrument has a range $R_1$ of measurement between -12D and +8D (the range of measurement is determined mainly by first-order aberrations, i.e., defocus), an ACD capable of inducing a defocus of -6D will shift the range $R_1$ to a new range $R_2$ of measurement up to -18D (i.e., range $R_2$ provides a range of measurement from -18D to +2D). Similarly, an ACD capable of inducing a defocus of +6D will shift the range $R_1$ to a new range $R_3$, up to +14D (i.e., range $R_3$ provides a range of measurement from -6D to +14D).

Another embodiment of the method and system for Aberroscope calibration and discrete compensation of the present invention includes providing continuous compensation using adaptive optical elements, such as a liquid crystal SLM, a MEMS device, or a continuous membrane deformable mirror. Continuous wavefront compensation/correction is very useful in some applications related to detecting and measuring functional vision.

The accuracy of a wavefront aberration measurement in a Hartmann-Shack analyzer is in part determined by the spot size produced by a lenslet on the detector plane and by the separation between two adjacent spots. These factors depend upon a number of parameters, including, in the case of an eye wavefront measurement, the spot size produced by the retinal probe beam, $\delta_{retina}$.

Pupil diameter and pupil magnification are also important. For example, with reference to the optical train **10** of FIGURE 1, the pupil magnification of the wavefront measurement instrument is given by:

$$M_{pupil} = (f_2/f_1) \times (f_4/f_3)$$

In the case of the optical train **30** of FIGURE 2, the pupil magnification becomes:

$$M_{Pupil} = f_4/f_1, \text{ because } f_2 = f_3$$

Also important are the lenslet array effective focal length, denoted as $f_5$ in FIGURES 1 and 2, the clear aperture of a lenslet, and the eye's aberration.

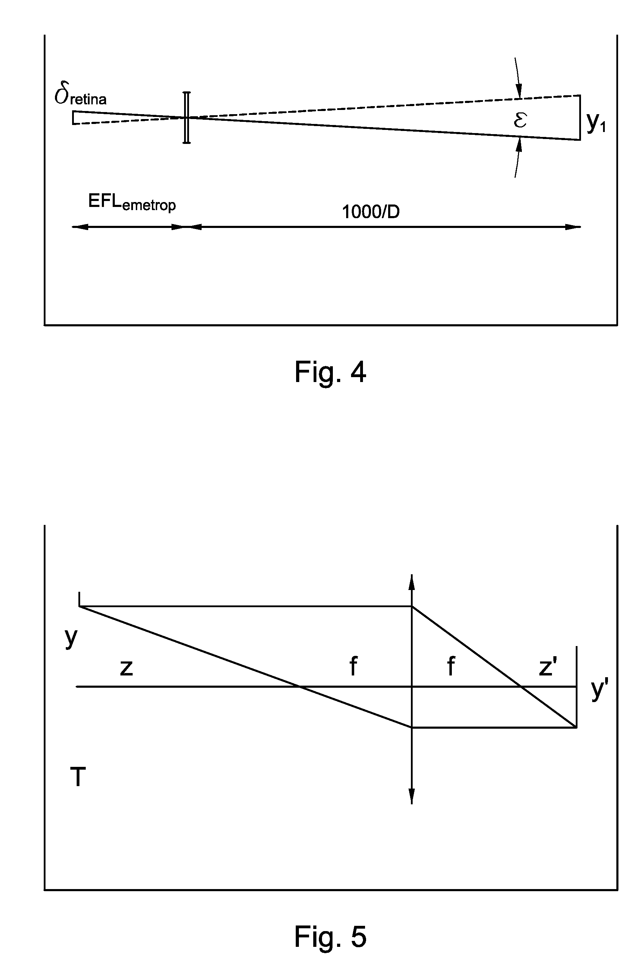

A paraxial approach to calculating the spot size at the lenslet array focal plane will be shown with reference to FIGURES 4-7. From the retina to the object space (FIGURE 4):

$$\frac{y_1}{\delta_{retina}} = \frac{1000}{D \times EFL_{emetrop}} \qquad \text{(Equation 1)}$$

The angle subtended by $\delta_{retina}$ is:

$$\varepsilon = \frac{\delta_{retina}}{EFL_{emetrop}} \qquad \text{(Equation 2)}$$

Newton's equations show that (FIGURE 5):

$$Z \times Z' = -f^2 \qquad \text{(Equation 3)}$$

and

$$\frac{y'}{y} = \frac{Z'}{f} = \frac{f}{Z} \qquad \text{(Equation 4)}$$

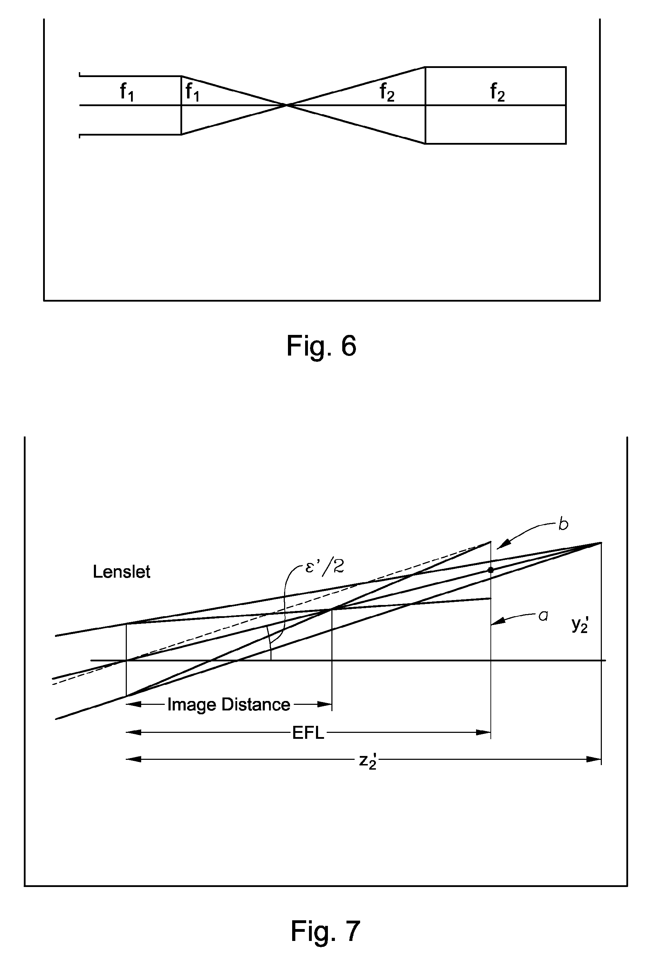

For an afocal relay system, the pupil magnification is (FIGURE 6):

$$M_{Pupil} = f_2/f_1 \qquad \text{(Equation 5)}$$

The angular magnification between pupils is:

$$\varepsilon' = \varepsilon/ = 1/ M_{Pupil} \qquad \text{(Equation 6)}$$

Applying Newton's equations to the first and the second lenses, one obtains:

$$\text{(Equation 7)}$$

$$z_1 \times z_1' = -f_1^2 ; \quad z_2 \times z_2' = -f_2^2 \quad \xrightarrow{z_1 \equiv 100/D,\ z_1' \equiv z_2} \quad z_2' = \frac{100}{D} \times (M_{Pupil})^2$$

and

$$\frac{y_2'}{y_1} = \frac{y_2'}{y_2} \times \frac{y_1'}{y_1} = \frac{z_1'}{f_1} \times \frac{f_2}{z_2} \quad \xrightarrow{z_1' \equiv z_2} \quad \frac{y_2'}{y_1} = M_{pupil} \qquad \text{(Equation 8)}$$

The spot size in the lenslet focal plane may be calculated as (FIGURE 7):

$$\delta = 2(a+b) = EFL \times \varepsilon' + EFL \times \frac{d}{|z_2'|} \qquad \text{(Equation 9)}$$

where d is the lenslet size.

Equations 2, 6, and 7 then yield:

$$\delta = \delta_a + \delta_b \qquad \text{(Equation 10)}$$

where:

$$\delta_a = EFL \times \frac{\delta_{retina}}{EFL_{emetrop} \times M_{pupil}}, \qquad \text{(Equation 11)}$$

represents the retinal probe beam spot contribution to the spot size in the lenslet focal plane, and where:

$$\delta_b = EFL \times \frac{d \times |D|}{1000 \times M_{pupil}^2} \qquad \text{(Equation 12)}$$

is the refractive ocular error contribution.

If an ACD is introduced into the optical path, whether in an intermediate position or in front of the lenslet array, the eye aberration can be dramatically reduced, and by consequences the spot size on the detector plane, thereby improving spot separation at the detector.

Several example calculations of an ACD are presented below.

**Example 1.** Lenses inducing pure defocus:

| Lens type | Power at 820 nm | Clear aperture | Radius of curvature | Conic constant | Thickness on axis | Glass |
|---|---|---|---|---|---|---|
| Piano-concave | -10D | 12 mm | -51.04 mm | -0.5865 | 1.5 mm | BK7 |
| Piano-convex | +10D | 12 mm | 51.04 mm | 0.58215 | 3 mm | BK7 |

**Example 2.** Computer-generated holograms inducing spherical aberration:

| ACD type | Clear aperture (mm) | $\phi = A_2\rho^2 + A_4\rho^4$; phase eq. of the Binary Surface (rad) | Induced aberration in Terms of Zernike Polynomials ($\mu$m) |
|---|---|---|---|
| Negative spherical aberration | 12 | $A_2=377$ <br> $A_4=-377$ | $C_4^0=-8.2$ |
| Positive spherical aberration | 12 | $A_2=-377$ <br> $A_4=377$ | $C_4^0=+8.2$ |

where $\rho$ is the normalized radial aperture coordinate and the wavefront equation is $WF = C_4^0(6\rho^4-6\rho^2-1)$.

**Example 3.** Computer-generated hologram inducing pure coma: For a clear aperture: 12 mm and $\lambda=0.8$ $\mu$m, the phase equation of the binary surface (in radians) is:

(Equation 13)

$$\Phi(X,Y) = \frac{A_4}{r_M{}^4} * \{[X^2 + (Y + Y_0)^2]^2 - [X^2 + (Y - Y_0)^2]^2 - 8\,y_0{}^3\,Y\}$$

where $A_4$=202; $r_M$=6 mm, and $y_0$=0.7 mm. The induced aberration in terms of Zernike polynomials is (in μm):

$$WF\,(X,Y) = \frac{3C_3{}^{-1}}{r_M{}^2}\,(X^2Y+Y^3)\,, \qquad \text{(Equation 14)}$$

where $C_3{}^{-1}$ = -8.2.

Using commercial optical-design software, the CGH can be modeled as a diffractive surface defined by a phase function. The phase function is specified by an equation that could be a radial or Cartesian polynomial, a Zernike polynomial, or a Sweatt model phase equation. Basically, the optical function $\phi(x,y)$ is determined by ray tracing from the ocular wavefront to be generated to the focal point of the setup. As the CGH null has to work in the first diffraction order, a carrier frequency must be added to the phase function in such a way as to ensure the separation of the diffraction orders. The CGH will be appropriately titled or decentered with respect to the aberroscope axis.

Photolithography is probably the most commonly used technique for making CGHs. Binary optics provides three main advantages: the capability of producing complex diffractive structures; low production cost for two-phase-level elements; and the possibility of high diffraction efficiency with multilevel elements.

The phase function $\varphi(x,y)$ reproduced by a diffractive binary element is wrapped to an interval between 0 and an integral multiple of $2\pi$. The phase profile is given by:

$$\psi(x,y)=[\phi(x,y)+\phi_0]\mathrm{mod}2\pi, \qquad \text{(Equation 15)}$$

where $\varphi_0$ is a constant phase offset. The two-level binary phase functions can be described in various modes, for instance, $\Psi_{binary}(x) = \pi$, when $\tfrac{1}{2}(x_k + X_{k+1}) \le x \le \tfrac{1}{2}(X_{k+1} + x_{k+1})$, $k\,\varepsilon\,Z$; and $\Psi_{binary}(x) = 0$, when $x\,\varepsilon$ rest of $R$, where $x_k$ and $X_k$ are the solutions of the equations $\varphi(x,y) = 2k\pi$ and $\varphi(x,y) = (2k-1)\pi$, respectively.

The surface-relief profile $h(x,y)$ for a CGH etched on a substrate with a refractive index $n$ is given by:

$$h(x,y)=\frac{\lambda}{2\pi}\times\frac{\Psi_{binar}(x\text{-}y)}{n\text{-}1} \qquad \text{(Equation 16)}$$

Typically, a CGH is designed to operate at a specific wavelength.

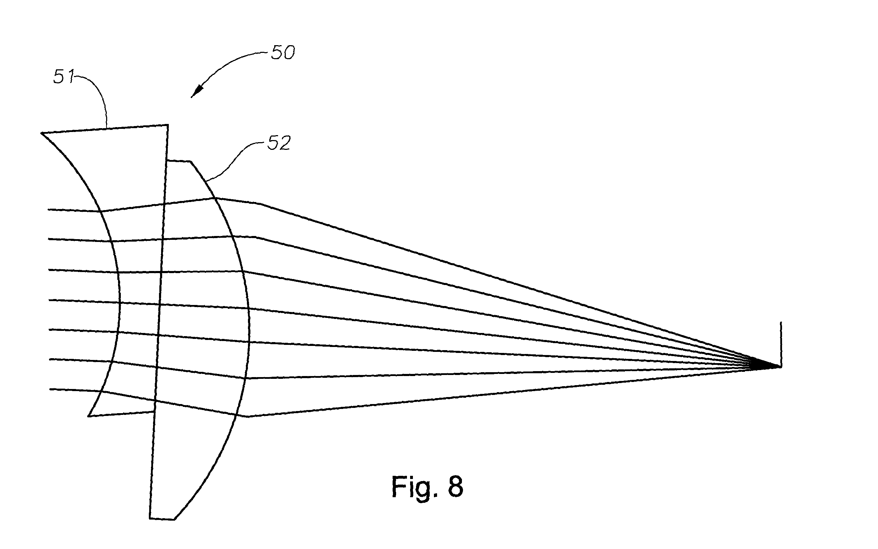

**Example 4.** Combination of two lenses producing a known aberration. FIGURE 8 illustrates a doublet **50** having the following specification: Entrance pupil diameter = 10 mm. The two elements are decentered ±2 mm with respect to the optical axis. The first element **51** is a piano-concave lens with a radius of curvature of 12.55 mm, a conic constant of 0.17, and a wedge (tilt) of 12°. The second element **52** is a piano-convex lens with a biconic convex surface having radii of curvature in two perpendicular planes of 15.23 mm and 15.05 mm. The wavefront aberration produced by this doublet expressed in terms of Zernike polynomials is:

$$WF(\rho,\theta)=C_3{}^{-1}(3\rho^3\text{-}2\rho)\sin\theta+C_4{}^0(6\rho^4\text{-}6\rho^2+1), \qquad \text{(Equation 17)}$$

wherein the first term represents coma and the second, spherical aberration; $C_3{}^{-1}$=7.94; $C_4{}^0$= -0.234; and $\rho$ is the normalized radial aperture coordinate.

**Example 5.** Combination of two CGHs. In this example, a "doublet" of two CGHs provides a specified amount of coma *without* spherical aberration, a unique feature. For a clear aperture of 12 mm and $\lambda$=0.82 μm, the two CGHs that are decentered ±0.7 mm with respect to the optical axis have phase equations (in rad) given by:

$$\phi_1(r) = -0.164 \times r^4 \qquad \text{(Equation 18)}$$

$$\phi_2\,(r) = +0.164 \times r^4 \qquad \text{(Equation 19)}$$

where $r$ is the radial coordinate (in mm). The induced aberration in terms of Zernike polynomials is (in $\mu$m):

$$WF(X,Y)=\frac{3\,C_3^{-1}}{r_M^2}\,(X^2Y+Y^3)$$

where $C_3^{-1} = -8.65$, $r_M = 6$ mm; and X and Y are pupil coordinates (in mm).

A tolerance analysis related to the axial and transverse positioning of the ACD can show the sensitivity to alignment/positioning parameters. Such an analysis has been performed by the present inventor ("Ocular Aberrations Induced by Centration Errors in Waveguided Treatments," The Association for Research in Vision and Ophthalmology ("ARVO"), 2002).

Validation can be performed using an interferometric setup for any ACD standalone, as well as for an entire wavefront measurement instrument including the ACD. In this case, a flat mirror can be placed in front of the lenslet array in order to test the system in double-pass mode.

It may be appreciated by one of average skill in the art that the present invention confers the benefit of improved wavefront accuracy. Further, compared with classical lenses that can generate limited types of ocular aberration, such as defocus error and spherical aberration, a computer-generated hologram can in principle reproduce any individual ocular aberration or a combination of different aberrations to generate a composite wavefront.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiments thereof, and the advantageous new and useful results obtained thereby, the new and useful constructions, and reasonable mechanical equivalents thereof now obvious to those of average skill in the art, are set forth in the appended claims.

## Claims

1. A device (10,30) for calibrating an aberroscope comprising an optical element (40,50) insertable into an optical path of a wavefront (14,35) analyzer, the optical element adapted to induce a predetermined aberration in a wavefront for presentation to the wavefront analyzer.

2. The device recited in Claim 1, wherein the optical element (40) comprises a hologram.

3. The device recited in Claim 2, wherein the hologram comprises a substrate having a surface imposed thereon adapted to reproduce a desired optical wavefront, the desired optical wavefront having the predetermined aberration.

4. The device recited in Claim 3, wherein the desired aberrated wavefront comprises a wavefront modeled using Zernike polynomials.

5. The device recited in Claim 2, wherein the hologram comprises a computer-generated hologram.

6. The device recited in Claim 2, wherein the hologram is insertable into an optical path of a Hartmann-Shack wavefront analyzer.

7. The device recited in Claim 1, wherein the optical element (50) comprises a lens optimized for a specific power and aberration.

8. The device recited in Claim 7, wherein the lens comprises a plurality of lenses (51,52) operating in concert to induce the predetermined aberration.

9. The device recited in Claim 1, wherein the optical element (50) comprises a spatial light modulator adapted to induce the predetermined aberration.

10. The device recited in Claim 1, wherein the optical element comprises means for inducing a predetermined amount

**EP 1 533 647 A1**

of defocus in the wavefront, the defocus amount serving to shift a wavefront analyzer range of measurement from a first range between a first minimum value and a first maximum value to a second range between a second minimum value and a second maximum value, the first and the second minimum value and the first and the second maximum value differing by the predetermined amount of defocus.

11. The device recited in Claim 1, wherein the optical element is selected from a group consisting of a reflective (40) and a transmissive (50) optical element.

12. A system (10,30) for calibrating an aberroscope, comprising:

   a wavefront (14,35) analyzer comprising a wavefront detector (12) at a downstream end of an optical path; and an optical element (40,50) insertable into an optical path of the wavefront analyzer, the optical element adapted to induce a predetermined aberration in a wavefront for presentation to the analyzer.

13. The system recited in Claim 12, further comprising means for collimating (17,23,33,42) an incoming wavefront onto the detector (12) downstream of the optical element.

14. The system recited in Claim 12, wherein the optical element is selected from a group consisting of a reflective (40) and a transmissive (50) optical element.

15. The system recited in Claim 12, wherein the optical element (40) comprises a hologram.

16. The system recited in Claim 15, wherein the hologram comprises a computer-generated hologram.

17. The system recited in Claim 12, wherein the wavefront analyzer comprises a Hartman-Shack wavefront analyzer.

18. The system recited in Claim 17, wherein the Hartmann-Shack wavefront analyzer comprises:

   an entrance pupil (13,31) for admitting the incoming wavefront (14);
   a first afocal optical system (15,100) for forming an image of the entrance pupil onto an intermediate pupil plane (19,39);
   a lenslet array (11); and
   a second afocal optical system (20) for forming an image of the intermediate pupil plane onto the lenslet array, the lenslet array for sampling the intermediate pupil image onto the wavefront detector; and wherein:

      the optical element (40,50) is positioned at a location selected from a group consisting of adjacent the entrance pupil, at the intermediate pupil plane, and adjacent a plane of the lenslet array.

19. The system recited in Claim 18, further comprising a beamsplitter (41), and wherein:

   the optical element (40) comprises a reflective computer-generated hologram;
   the first afocal optical system (100) comprises a first converging lens (32) and a first collimating lens (33), the first collimating lens positioned to receive the incoming wavefront (35) from the first converging lens at a first face (36) and to output a collimated wavefront (37) from a second face (38), the first converging lens and the first collimating lens together operating to image the entrance pupil (31) onto the intermediate pupil plane (39), the computer-generated hologram positioned to receive and reflect the collimated wavefront onto the first collimating lens, the beamsplitter (41) positioned and adapted to permit the incoming wavefront (35) exiting the first converging lens (32) to pass through substantially unaltered;
   the second afocal optical system comprises a second converging lens, the second converging lens comprising the first collimating lens (33) positioned to receive a reflected wavefront (37') from the hologram at the second face and to output a converging wavefront from the first face onto the beamsplitter; and
   the second afocal optical system further comprises a second collimating lens (42) positioned to receive the converging wavefront from the beamsplitter and to output a second collimated wavefront (43) onto the lenslet array (11).

20. A method for calibrating an aberroscope comprising the steps of:

   passing an unaberrated wavefront (14,35) along an optical path leading to a wavefront analyzer;

9

inducing a predetermined aberration in the unaberrated wavefront to form an aberrated wavefront using an optical element (40,50) positioned in the optical path upstream of a wavefront analyzer;
analyzing the aberrated wavefront exiting the optical element using the wavefront analyzer; and
calibrating the wavefront analyzer using data generated by the wavefront analyzer from the aberrated wavefront.

21. The method recited in Claim 20, wherein the inducing step comprises shifting a wavefront analyzer range of measurement from a first range between a first minimum value and a first maximum value to a second range between a second minimum value and a second maximum value, the first and the second minimum value and the first and the second maximum value differing by an amount determined by the optical element (40,50).

22. The method recited in Claim 20, further comprising the step of collimating the aberrated wavefront onto the wavefront analyzer downstream of the optical element (40,50).

23. The method recited in Claim 20, further comprising the steps of:

admitting the unaberrated wavefront into an entrance pupil (13,31);
using a first afocal optical system (15,100) to form an image of the entrance pupil onto an intermediate pupil plane (19,39);
using a second afocal optical system (20) to form an image of the intermediate pupil plane onto a lenslet array (11) of the wavefront analyzer; and
sampling the intermediate pupil plane image at the lenslet array and presenting the image samples onto the wavefront analyzer; and wherein:

the aberration inducing step comprises positioning the optical element (40,50) at a location selected from a group consisting of adjacent the entrance pupil, at the intermediate pupil plane, and adjacent a plane of the lenslet array.

24. The method recited in Claim 23, wherein:

the optical element (40) comprises a reflective computer-generated hologram;
the first afocal optical system (100) comprises a first converging lens (32) and a first collimating lens (33), wherein the first collimated lens is positioned to receive a first wavefront (35) from the first converging lens at a first face (36) and to output a collimated, unaberrated wavefront (37) from a second face (38); and
the inducing a predetermined aberration step comprises;
receiving the collimated, unaberrated wavefront at the hologram;
inducing the predetermined aberration in the collimated, unaberrated wavefront to produce an aberrated wavefront (37'); and
reflecting the aberrated wavefront onto the first collimating lens (33); and
further comprising the steps of;
converging the aberrated wavefront by passing through the second face and out of the first face of the first collimating lens; and
reflecting the converged, aberrated wavefront onto a second collimating lens (42) to output a collimated, aberrated wavefront (43) onto the lenslet array (11).

25. The method recited in Claim 20, wherein the optical element (50) comprises means for inducing a predetermined amount of defocus in the unaberrated wavefront, the defocus amount serving to shift a wavefront analyzer range of measurement from a first range between a first minimum value and a first maximum value to a second range between a second minimum value and a second maximum value, the first and the second minimum value and the first and the second maximum value differing by the predetermined amount of defocus.

26. A method of constructing a device (10,30) for calibrating an aberroscope comprising the steps of:

determining a desired aberration;
creating a hologram having the desired aberration; and
positioning the hologram upstream of a wavefront analyzer.

27. The method recited in Claim 26, wherein the determining step comprises modeling a wavefront having the desired aberration using Zernike polynomials.

**28.** The method recited in Claim 26, wherein the determining and creating steps comprise using a computer to calculate the desired aberration and to create the hologram on a substrate.

**29.** The method recited in Claim 26, wherein the hologram comprises one of a transmissive and a reflective computer-generated hologram.

Fig. 1

Fig. 2

Fig. 3

Fig. 4

Fig. 5

Fig. 6

Fig. 7

Fig. 8

# EP 1 533 647 A1

**European Patent Office**

## EUROPEAN SEARCH REPORT

Application Number

EP 04 10 5850

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 379 005 B1 (WILLIAMS DAVID R ET AL) 30 April 2002 (2002-04-30) <br> * figures * <br> * column 4, line 26 - column 8, line 11 * <br> ----- | 1-6,9-27 | G02B27/44 <br> A61B3/10 |
| X | US 6 086 204 A (MAGNANTE ET AL) 11 July 2000 (2000-07-11) <br><br> * figures 1,2 * <br> * column 5, line 40 - column 7, line 3 * <br> ----- | 1,6-8, 11-14, 17,18 | |
| X | JOHANNES PFUND, NORBERT LINDLEIN, JOHANNES SCHWIDER: "Misalignment effects of the Shack-Hartmann sensor" APPLIED OPTICS, vol. 37, no. 1, 1 January 1998 (1998-01-01), pages 22-27, XP002322182 <br> * the whole document * <br> ----- | 1,6,12, 20 | |
| X | US 5 936 720 A (NEAL ET AL) 10 August 1999 (1999-08-10) <br><br> * claims 15,16,18,33-35 * <br> * column 3, line 25 - line 30 * <br> * figures 1,2,15,16 * <br> * column 10, line 12 - line 28 * <br> ----- | 1,6-8, 10-14, 17,20-22 | TECHNICAL FIELDS SEARCHED (Int.Cl.7) <br> A61B <br> G02B |
| X | US 2003/112411 A1 (MARTINO RONALD J) 19 June 2003 (2003-06-19) <br><br> * paragraph [0007] * <br> * paragraph [0017] - paragraph [0032] * <br> * figures * <br> ----- | 1,6-14, 17, 20-22,25 | |

The present search report has been drawn up for all claims

| Place of search | Date of completion of the search | Examiner |
|---|---|---|
| Munich | 23 March 2005 | Windecker, R |

EPO FORM 1503 03.82 (P04C01)

**ANNEX TO THE EUROPEAN SEARCH REPORT
ON EUROPEAN PATENT APPLICATION NO.**   EP 04 10 5850

This annex lists the patent family members relating to the patent documents cited in the above-mentioned European search report.
The members are as contained in the European Patent Office EDP file on
The European Patent Office is in no way liable for these particulars which are merely given for the purpose of information.

23-03-2005

| Patent document cited in search report | | Publication date | Patent family member(s) | | Publication date |
|---|---|---|---|---|---|
| US 6379005 | B1 | 30-04-2002 | US | 6095651 A | 01-08-2000 |
| | | | US | 5949521 A | 07-09-1999 |
| | | | US | 5777719 A | 07-07-1998 |
| | | | US | 2003025874 A1 | 06-02-2003 |
| | | | AU | 723645 B2 | 31-08-2000 |
| | | | AU | 5806298 A | 17-07-1998 |
| | | | BR | 9714178 A | 29-02-2000 |
| | | | CA | 2275762 A1 | 02-07-1998 |
| | | | CN | 1245406 A | 23-02-2000 |
| | | | EP | 0969760 A1 | 12-01-2000 |
| | | | JP | 2001507258 T | 05-06-2001 |
| | | | WO | 9827863 A1 | 02-07-1998 |
| US 6086204 | A | 11-07-2000 | EP | 1215992 A1 | 26-06-2002 |
| | | | JP | 2003509731 T | 11-03-2003 |
| | | | WO | 0121061 A1 | 29-03-2001 |
| US 5936720 | A | 10-08-1999 | US | 5864381 A | 26-01-1999 |
| | | | US | 6052180 A | 18-04-2000 |
| | | | US | 6130419 A | 10-10-2000 |
| US 2003112411 | A1 | 19-06-2003 | AU | 2002366342 A1 | 30-06-2003 |
| | | | CA | 2469691 A1 | 26-06-2003 |
| | | | EP | 1455639 A1 | 15-09-2004 |
| | | | WO | 03051190 A1 | 26-06-2003 |

EPO FORM P0459

For more details about this annex : see Official Journal of the European Patent Office, No. 12/82